# EUROPEAN PATENT APPLICATION

(11) **EP 4 129 360 A1**
(43) Date of publication of application: **08.02.2023**
(21) Application number: 21779753.9
(22) Date of filing: 31.03.2021
(51) Int. Cl.: A61M 5/00, A61M 5/31, A61M 5/34, A61M 1/16

(54) **MEDICAL SYRINGE BARREL, AND PACKAGING FOR SAME**

(30) Priority: 31.03.2020 JP 2020062641
(71) Applicant: Daikyo Seiko, LTD., Sano-shi, Tochigi 327-0813 (JP)
(72) Inventor: YOSHIDA, Takayuki, Sano-shi, Tochigi 327-0813 (JP)
(74) Representative: Grünecker Patent- und Rechtsanwälte PartG mbB
(86) International application number: PCT/JP2021/013775
(87) International publication number: WO 2021/201074

(57) **Abstract**

**Problem**

To provide a medical syringe barrel and a package thereof which secures a sufficient stroke even for operating a Lure Lock type small capacity syringe, restrains rattle of the syringe barrel inside the tubular member, prevents displacement and makes the drawing of the syringe barrel from the tubular member easier more effectively at the time of transporting the syringe barrel while holding the syringe barrel in the tubular member of the nest.

Solution

A medical syringe barrel comprising a tubular barrel section for accommodating an internal-use solution in the interior thereof, a tubular injection nozzle section formed at one end of the barrel section, and a flange section formed at an opening section on the other end of the barrel section to project radially outward, wherein the barrel section includes an intermediate body part having an outer diameter which is smaller than the outer diameter of the injection nozzle section and a large outer diameter barrel section having an outer diameter which is equal to the outer diameter of the injection nozzle section and the large outer diameter barrel section is formed between the flange section and the intermediate body part.

## Description

### Technical Field

The present invention relates to a medical syringe barrel for constituting a medical syringe and a package for transporting it.

### Background Art

In general, a medial syringe comprises a syringe barrel having a tip on which a needle is to be attached and a syringe plunger which is to be inserted into the syringe barrel from an opening on the other end for moving a piston in the axial direction. A flange is formed at the opening on the other end of the syringe barrel to project radially outward. There are the Lure Tip type, the Lure Lock type or the like as a type of a syringe tip portion to which an injection needle is to be attached. The structure for fixing an injection needle is determined by the ISO standards in view of medical accident prevention. The Lure Lock type is a type of having a locking function at a syringe tip portion and it is suitable for connecting with an injection needle firmly.

Although the diameter of a Lure Lock part as the tip portion of a syringe barrel is determined by the ISO standards, it is necessary to reduce the inner diameter of the barrel section in which an internal-use solution is filled in a minimal capacity syringe in view of necessity of keeping a predetermined stroke for the sake of being easy to use at the time of injection. When a syringe barrel having an outer diameter of a barrel section which is equal to the diameter of a Lure Lock section is manufactured by injection molding of a thermoplastic resin material, it causes a molding defect in the barrel section having an increased wall thickness. For this reason, as shown in Patent Document 1, a barrel was proposed for keeping a sufficient stroke by making the outer diameter of a barrel section smaller than the diameter of the Lure Lock section. Patent Document 2 discloses a structure of a syringe barrel comprising a large outer diameter section at the end edge side which has an inner diameter larger than the inner diameter of an intermediate section.

For simplifying the medicine management, controlling the infection, preventing errors at the time of dispensing, speeding up the administration and so on, a prefilled syringe, in which an accurate dosage of medicine is previously filled, is used. In general, the components for assembling a syringe like a syringe barrel, a piston and a plunger are transported to a pharmaceutical company, and it is filled with an internal-use solution and a syringe is assembled. Therefore, it is necessary to transport a syringe keeping a sterilized state, and it is proposed to use a container comprising a nest and a tub as shown in Patent Documents 3 and 4 as an example.

Patent Document 1: Japanese laid-open patent application 2013-116289
Patent Document 2: Japanese design registration No. 1468280
Patent Document 3: International laid-open patent application WO2014/109011A1
Patent Document 4: Japanese patent No. 6040025

### Disclosure of Invention

### Problems to be resolved by the Invention

When a syringe barrel shown in Patent Document 1 or 2 is stored in a nest shown in Patent Document 3 or 4 for transporting the syringe barrel together with other components like a piston and a plunger to a pharmaceutical company for assembling a syringe as a prefilled syringe, a rattle is generated in the nest at the time of transportation. There is a possibility that the rattle causes a breakage by bringing members into contact mutually during the transportation, and it may cause trouble when the syringe barrel is drawn from the nest.

Therefore, it is desired to provide a syringe barrel and a package thereof which can prevent the members from undesirable mutual contact at the time of transporting them in a nest and drawing from the nest and thereby realize easy drawing operation.

### Means for solving the Problems

(1)A medical syringe barrel according to the present invention comprises a tubular barrel section for accommodating an internal-use solution in the interior thereof, an injection nozzle section formed at one end of the barrel section, and a flange section formed at an opening section on the other end of the barrel section to project radially outward.

The barrel section includes an intermediate body part having an outer diameter which is smaller than the outer diameter of the injection nozzle section and a large outer diameter barrel section having an outer diameter which is equal to the outer diameter of the injection nozzle section. The large outer diameter barrel section is formed between the flange section and the intermediate body part.

When a medical syringe barrel configured as described above is inserted into a tubular member of a nest from above taking the syringe barrel with the injection nozzle section facing down, the injection nozzle section having an outer diameter which is slightly smaller than the inner diameter of the tubular member of the nest passes through the tubular member, the intermediate body part and the large outer diameter barrel section are inserted into the tubular member in this order, and then the syringe barrel is stored in the tubular member by bringing the flange section into contact with the upper end of the tubular member.

By setting the outer diameter of the large outer diameter barrel section equal to the outer diameter of the injection nozzle section, it becomes possible to prevent rattle of the syringe barrel in the tubular member when the syringe barrel is transported by storing in the nest. It becomes hard to cause displacement because the center axis of the tubular member coincides with the center axis of the syringe barrel. It also makes easy to draw the syringe barrel from the tubular member in a straight line.

(2) In a medical syringe barrel according to the present invention described in (1), the axial length of the large outer diameter barrel section is set to 1/3 or less of the axial length of the barrel section.

By setting the length of the large outer diameter barrel section to an appropriate value, it is possible to restrain rattle of the syringe barrel inside the tubular member, prevent displacement and make the drawing of the syringe barrel from the tubular member easier more effectively. On the other hand, if the large outer diameter barrel section is too long, there is an inconvenience that the distance between the opening at the flange section side of the syringe barrel and the piston default position (the predetermined content filled position) becomes long at the time of vacuum plugging of the piston. Therefore, it is preferable to set the axial length of the large outer diameter barrel section to 1/3 or less of the length of the barrel section.

(3)A package of a medical syringe barrel according to the present invention comprises a nest including a flat plate member and a tubular member, and at least one syringe barrel being held in the nest, the flat plate has at least one opening, one end of the tubular member is connected to the opening and the other end of the tubular member protrudes upward from the flat plate member.

The syringe barrel held in the nest includes a tubular barrel section for accommodating an internal-use solution in the interior thereof, an injection nozzle section formed at one end of the barrel section, and a flange section formed at an opening section on the other end of the barrel section to project radially outward. The barrel section includes an intermediate body part having an outer diameter which is smaller than the outer diameter of the injection nozzle section and a large diameter barrel section having an outer diameter which is equal to the outer diameter of the injection nozzle section.

The inner diameter of the tubular member is set to a value which is larger than the outer diameter of the large outer diameter barrel section and smaller than the outer diameter of the flange section. The flange section is in contact with the protruding end of the tubular member, and at least a portion of the barrel section is held inside the tubular member.

When a medical syringe barrel configured as described above is inserted into a tubular member of a nest from above taking the syringe barrel with the injection nozzle section facing down, the injection nozzle section having an outer diameter which is slightly smaller than the inner diameter of the tubular member of the nest passes through the tubular member, the intermediate body part and the large outer diameter barrel section are inserted into the tubular member in this order, and then the syringe barrel is stored in the tubular member by bringing the flange section into contact with the upper end of the tubular member.

By setting the outer diameter of the large outer diameter barrel section to be equal to the outer diameter of the injection nozzle section, it becomes possible to prevent rattle of the syringe barrel inside the tubular member when the syringe barrel is transported. It becomes hard to cause displacement because the center axis of the tubular member coincides with the center axis of the syringe barrel. It also makes easy to draw the syringe barrel from the tubular member in a straight line.

(4) In a package of a medical syringe barrel according to the present invention described in (3), the axial length of the large outer diameter barrel section is set to 1/3 or less of the axial length of the barrel section.

By setting the length of the large outer diameter barrel section to an appropriate value, it is possible to restrain rattle of the syringe barrel inside the tubular member, prevent displacement and make the drawing of the syringe barrel from the tubular member easier more effectively. On the other hand, if the large outer diameter barrel section is too long, there is an inconvenience that the distance between the opening of the flange of the syringe barrel and the piston default position (the predetermined content filled position) becomes long at the time of vacuum plugging of the piston. Therefore, it is preferable to set the axial length of the large outer diameter barrel section to 1/3 or less of the axial length of the barrel section.

### Effect of Invention

According to a medical syringe barrel and a package thereof according to the present invention, it is possible to secure a sufficient stroke even for operating a Lure Lock type small capacity syringe, and it is possible to restrain rattle of the syringe barrel inside the tubular member, prevent displacement and make the drawing of the syringe barrel from the tubular member easier more effectively at the time of transporting the syringe barrel while holding the syringe barrel in the tubular member of the nest.

### Brief Description of Drawings

Fig. 1 (A) shows a perspective view of a configuration example of a medical syringe made by assembling a syringe barrel, in which an internal-use solution is filled, according to one embodiment of the present invention together with a piston, a plunger and a finger grip, and (B) shows a perspective view of a medical syringe shown in (A) which is observed from a different angle.
Fig.2 (A) shows an axial cross-sectional view of a syringe barrel according to one embodiment of the present invention, and (B) shows a side view observed from the arrow B in (A).
Fig. 3 shows a perspective view of a schematic configuration of a container used for a package of a medical syringe barrel according to one embodiment of the present invention.
Fig. 4 shows a partial cross-sectional view of a package of a medical syringe barrel according to one embodiment of the present invention.
Fig. 5 shows a perspective view of an exterior appearance of a package of a medical syringe barrel according to one embodiment of the present invention.
Fig. 6 shows an example of using a tiptoe of an arm for handling a syringe barrel in a package of a medical syringe barrel according to one embodiment of the present invention.

### A Mode for implementing the Invention

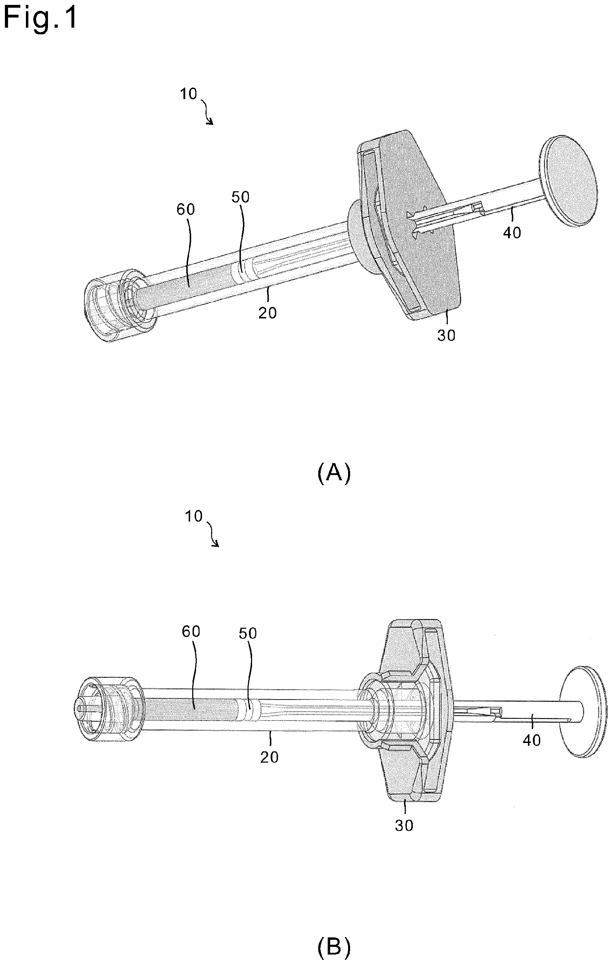

A configuration of a medical syringe barrel according to one embodiment of the present invention will be explained below referring to the drawings. Fig. 1 shows a perspective view of a configuration example of a medical syringe which is assembled by a syringe barrel according to one embodiment of the present invention, a piston, a plunger and a finger grip, in which the syringe barrel is filled with an internal-use solution. This medical syringe is a prefilled syringe with a minimal capacity for ophthalmic use as an example, and a disposable syringe for which a single time use is assumed.

In Fig.1, a syringe 10 is an assembly made of a syringe barrel 20, a finger grip 30, a syringe plunger 40 and a piston 50. An internal-use solution 60 is accommodated in a hollow barrel section of the tubular syringe barrel 20, and it is arranged so that the internal-use solution 60 is pushed out from the tip of the syringe barrel 20 by pushing the piston 50 with the tip of the syringe plunger 40.

As shown in Fig.1, a syringe barrel 20 has a transparent tubular body of a molded article made of glass or a polyolefin resin material as an example, and it is possible to visually recognize the internal-use solution 60, the piston 50 and a part of the syringe plunger 40 which are inside the hollow barrel section.

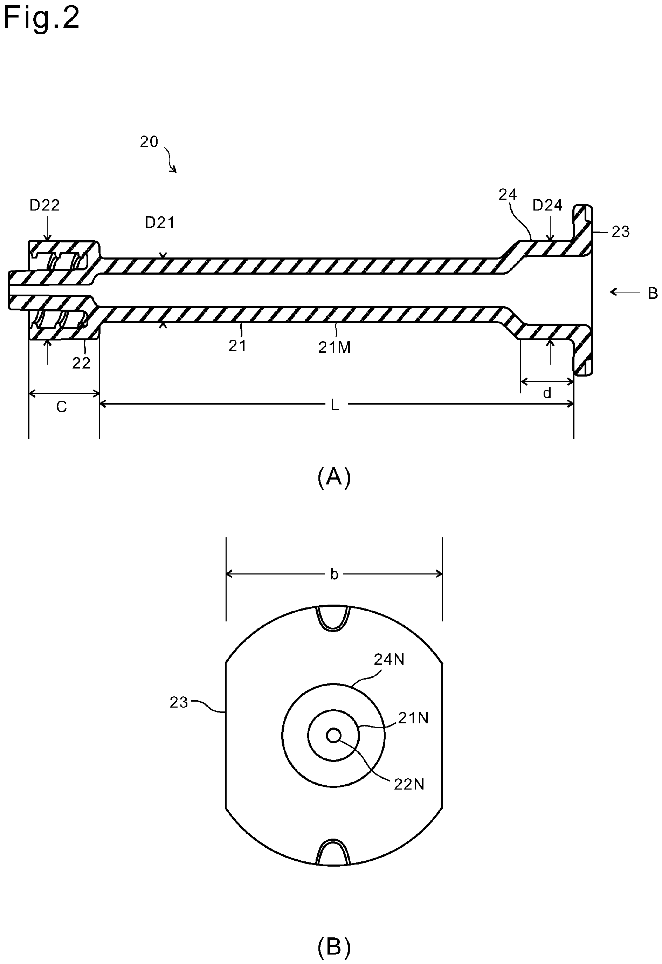

Fig. 2 is an axial cross-sectional view which shows a configuration of the syringe barrel 20 according to one embodiment of the present invention. The syringe barrel 20 is a molded article by injection molding of a synthetic resin material like a polyolefin resin material, and comprises a tubular barrel section 21 for accommodating an internal-use solution in the interior thereof, an injection nozzle section 22 which is formed at one end of the barrel section 21 and to which a needle as an example is to be attached, and a flange section 23 which is formed at an opening section on the other end of the barrel section 21 to project radially outward. The syringe barrel 20 is used for a minimal capacity syringe of 0.25m for ophthalmic use as an example.

In Fig, 2 (A), the injection nozzle section 22 is formed as a Lure Lock type and complied with the ISO 80369-7 standards, wherein a screw tube part is connected to the outer side of a cylindrical nozzle tube part, the cylindrical nozzle tube part has a tapered surface, which is formed to be thinner toward the tip, on the outer circumference, and the screw tube part has a male screw on the inner wall. In this structure, by fitting an injection needle to the injection nozzle section 22 firmly, it is possible to certainly prevent the attached injection needle from becoming loose or being dislocated from the injection nozzle section, when it is used in the medical field or the like. Such a syringe barrel 20 may be used for mixed injection to a drip infusion portion, a two-liquid blending syringe between syringes or the like.

The injection nozzle section 22 is complied with the International Standards ISO80369-7 for preventing misconnection, and the shape and so on are determined by the standards. In Fig.2, the axial length C of the screw tube part of the injection nozzle section 22 is 70% or longer of the outer diameter D22. As shown in Fig.2(B), the flange section 23 has a shape of connecting both ends of the two arcs by two straight lines respectively when the syringe barrel 20 is observed from the direction shown by the arrow B in Fig. 2(A). The inner wall 22N of the nozzle tubular part of the injection nozzle section 22, the inner wall 21N of the intermediate body part 21M and the inner wall 24N of the large outer diameter barrel section 24 are viewed in this order concentrically.

In a minimal capacity syringe of 0.25m for ophthalmic use as an example has a small inner diameter (the diameter of the inner wall 21N) of the barrel section (the intermediate body part 21 M), in which an internal-use solution is filled, in view of easy use at the time of injection and necessity for keeping a predetermined length of the stroke.

In a normal syringe barrel having an equal outer diameter through the range from the injection nozzle section to the end edge of the barrel section (immediately before the flange section), if the inner diameter of the intermediate body part is made smaller, it will cause an imperfect molding which is called as sink marks because the thickness of the barrel section becomes larger. In this embodiment, as the syringe barrel 20 is an injection molded article of a synthetic resin material, the thickness of the intermediate body part 21M is not made larger and the outer diameter D21 of the intermediate body part 21M is made smaller in order to prevent an imperfect molding like sink marks by injection molding.

A large outer diameter barrel section 24 having an outer diameter which is larger than the outer diameter of the intermediate body part 21M is formed between the flange section 23 and the intermediate body part 21M. The thickness of the large outer diameter barrel section 24 is set to be equal to the thickness of the intermediate body part 21M, and the inner diameter of the large outer diameter barrel section 24(the diameter of the inner wall 24N) is set to a larger value.

The outer diameter D24 of the large outer diameter barrel section 24 is set to a value which is equal to the outer diameter D22 of the screw tube part of the injection nozzle section 22. It is preferable to set the outer diameter D21 of the intermediate body part 21M to 50% or larger with respect to the outer diameter D22 of the injection nozzle section 22 or the outer diameter D24 of the large outer diameter barrel section 24. There is a continuous curved connection part between the flange section 23 and the large outer diameter barrel section 24. There is a tapered part between the large outer diameter barrel section 24 and the intermediate body part 21M.

The axial length "d" of the part of the large outer diameter barrel section 24 having an outer diameter which is equal to the outer diameter D22 of the injection nozzle section 22 is determined in consideration of the length of the intermediate body part which is required for making a minimal capacity syringe and the length of the intermediate body part 21M which is required for vacuum plugging of the piston. If the axial length "d" of the part of the large outer diameter barrel section 24 having an outer diameter which is equal to the outer diameter D22 of the injection nozzle section 22 is too long, there is an inconvenience that the distance between the opening and the piston default position (the predetermined content filled positron) becomes longer at the time of vacuum plugging of the piston. The piston default position will not be set in the portion which has a larger inner diameter than the inner diameter of the intermediate body part 21M for accommodating an internal-use solution.

Due to the above considerations, the axial length "d" of the portion of the large outer diameter barrel section 24 having an outer diameter which is equal to the outer diameter D22 of the injection nozzle section 22 is set to a value in the range of L/3 ≧ d > 0 in relation with the length L of the barrel section 21 of the syringe barrel 20.

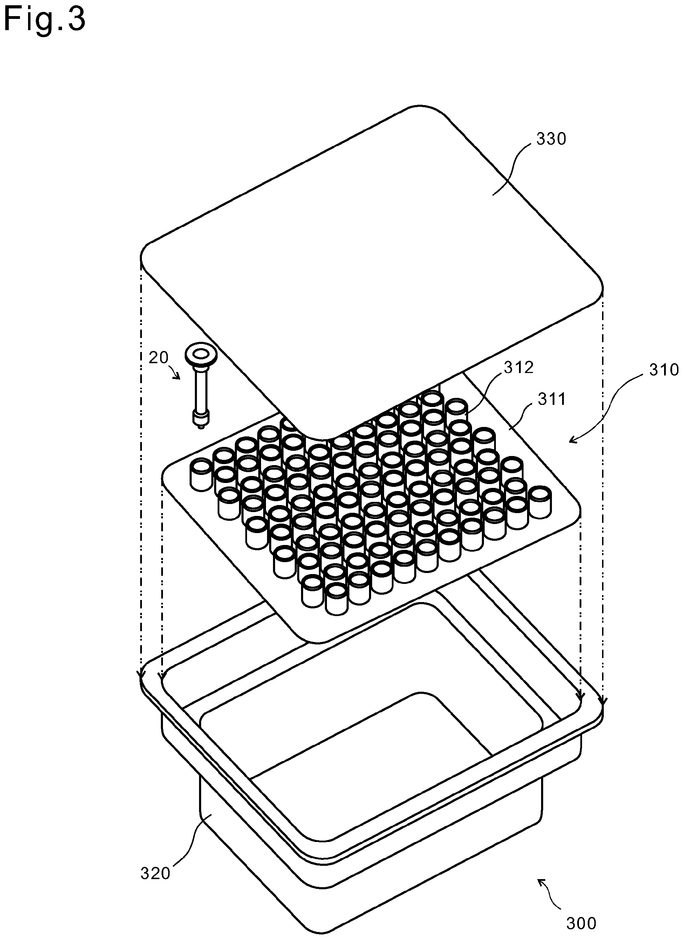

When a syringe barrel 20 configured as described above is transported to a pharmaceutical company or the like, a container for medical equipment, which comprises a nest and a tub shown in Fig.3, is used. A container 300 comprises a nest 310, a tub 320 and a film 330. The nest 310 comprises a flat plate member 311 and plural tubular members 312 which protrude upward from the flat plate member 311. The flat plate member 311 has plural openings, and one end of each of plural tubular members 312 is connected to each of the openings respectively, and the other ends of the plural tubular members protrude upward from the flat plate member 311. The inner diameter of each opening is equal to the inner diameter of each tubular member 312, and they are formed so that the center of each opening coincides with the center of each tubular member 312 respectively.

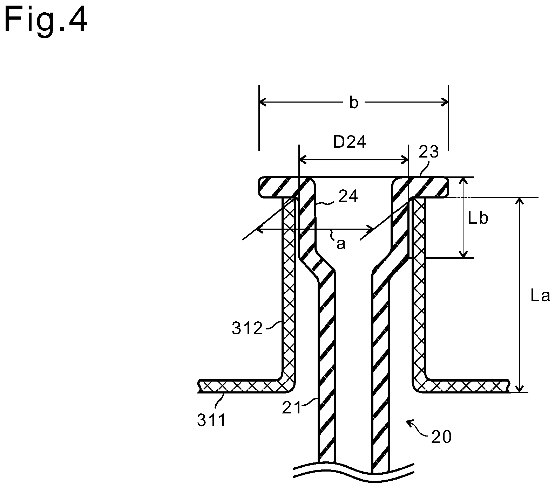

Each tubular member 312 functions as a holder for holding a syringe barrel 20. As shown in Fig.4, the inner diameter "a" of the tubular member 312 is set to be larger than the outer diameter D24 of the large outer diameter barrel section 24, the shortest outer diameter "b" of the flange section 23 is set to be larger than the inner diameter "a" of the tubular member 312. The flange section 23 is brought into contact with the protruding end of the tubular member 312, and a part of the barrel section 21 is held inside the tubular member 312.

In this embodiment, when the length "Lb" is a sum of the axial length "d" of the portion of the large outer diameter barrel section 24 which is equal to the outer diameter D22 of the injection nozzle section 22 and the thickness of the flange section 23, and the length "La" is a sum of the axial length of the tubular member 312 and the thickness of the flat plate member 311, it is preferable to set the length "Lb" to 40% through 60% of the length "La." The length "La" is determined by the ISO standards.

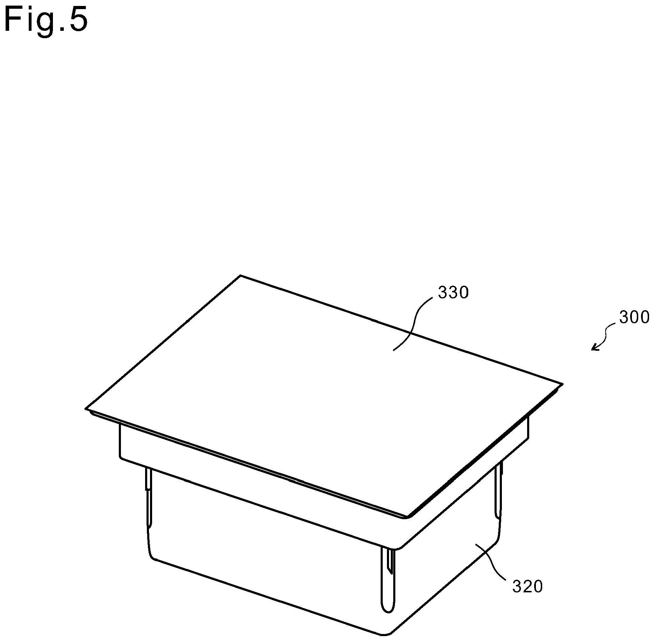

As shown in Fig.3, each of the plural syringe barrels 20 is held in each of the plural tubular members 312 in the nest 310 respectively and the nest 310 is stored in the tub 320. The tub 320 is sealed with a gas impermeable film 330 and then a medical syringe barrel package by the container 300 for medical equipment shown in Fig. 5 is obtained. The package is sterilized and transported to a pharmaceutical company or a medical institute, When the syringe barrel 20 is for a prefilled syringe, the container 300 can be opened at the pharmaceutical company or the medical institute, and the syringe barrel 20 can be filled with an internal-use solution immediately.

Other than the above-described package for sealing the opening portion with a gas impermeable film, the following package configurations are available, and these packaging methods and other known package configurations can be applied to the present embodiment.
(1) a method for packaging the entire tub under a reduced pressure condition after sealing the opening portion of the tub with a gas permeable film;
(2) a method for sealing the opening portion with a gas impermeable film under a reduced pressure condition after sealing the opening portion with a gas permeable film; and
(3) a packaging method for packaging and transporting keeping the cleanliness inside the tub at an appropriate control value which is different from the above-described methods.

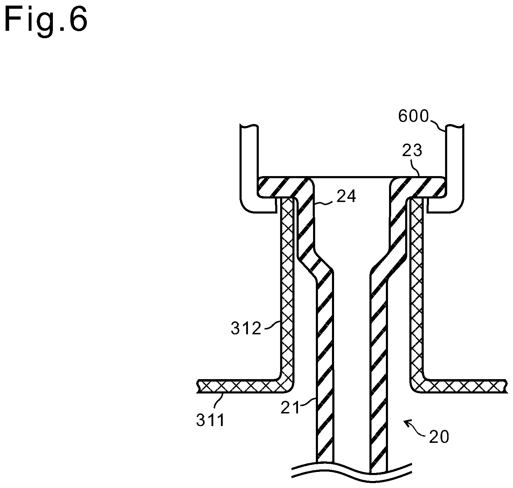

Next, the operation of a medical syringe barrel and a package thereof according to the present embodiment will be explained. The syringe barrel 20 having the shape shown in Fig.2 is produced by injection molding of a synthetic resin material, and the syringe barrel 20 is inserted into the tubular member 312 from above taking the syringe barrel 20 with the injection nozzle section 22 facing down by supporting the flange section 23 with the tiptoes 600 of the arm, and inserting the injection nozzle section 22, the intermediate body part 21M and the large outer diameter barrel section 24 into the tubular member 312 in this order, as shown in Fig.6. A part of the syringe barrel 20 is held by the tubular member 312 by supporting the flange section 23 by the protruding end of the tubular member 312.

As the outer diameter D22 of the injunction nozzle section 22 is set to be equal to the outer diameter D24 of the large outer diameter barrel section 24 and the inner diameter "a" of the tubular member 312 is set to be slightly larger than the outer diameters D22 and D24 of the two sections, the rattle is prevented inside the tubular member 312. When the syringe barrel 20 is transported to a pharmaceutical company in a condition of being stored in the container 300 shown in Fig.5 and filled with an internal-use solution, the syringe barrel 20 is pulled up by supporting the flange section 23 by the tiptoes 600 of the arm as shown in Fig.6.

At this moment, as the outer diameter D22 of the injection nozzle section 22 is equal to the outer diameter D24 of the large outer diameter barrel section 24, the syringe barrel 20 is pulled up in a condition that the center axis of the syringe barrel 20 approximately coincides with the center axis of the tubular member 312. By this configuration, the syringe barrel 20 can be drawn out from the tubular member 312 in a straight line.

Therefore, it is possible to prevent collision of the syringe barrel 20 with a member like the inner wall of the tubular member 312 because the operation of drawing out from the syringe barrel 20 becomes stable.

According to the embodiment described above, it is possible to provide a medical syringe barrel and a package thereof which secures a sufficient stroke for operation of a Lure Lock type small capacity syringe, prevents rattle during transportation by holding a syringe barrel inside a tubular member of a nest and makes the drawing out operation easy. A medical syringe barrel and a package thereof according to the present embodiment is preferable for a prefilled syringe which is assembled by filling an internal-use solution in a syringe barrel in advance.

### Explanation of References

- 10: syringe
- 20: syringe barrel
- 21: barrel section
- 21M: intermediate body part
- 22: injection nozzle section
- 23: flange section
- 24: large outer diameter barrel section
- 30: finger grip
- 40: syringe plunger
- 50: piston
- 60: internal-use solution
- 300: container
- 310: nest
- 311: flat plate member
- 312: tubular member
- 320: tub
- 330: film
- 600: tiptoe of arm

## Claims

1. A medical syringe barrel comprising a tubular barrel section for accommodating an internal-use solution in the interior thereof, an injection nozzle section formed at one end of the barrel section, and a flange section formed at an opening section on the other end of the barrel section to project radially outward, wherein
the barrel section includes an intermediate body part having an outer diameter which is smaller than the outer diameter of the injection nozzle section and a large outer diameter barrel section having an outer diameter which is equal to the outer diameter of the injection nozzle section; and
the large outer diameter barrel section is formed between the flange section and the intermediate body part.

2. A medical syringe barrel according to Claim 1, the axial length of the large outer diameter barrel section is set to be 1/3 or less of the axial length of the barrel section.

3. A package of a medical syringe barrel having a nest for holding at least one syringe barrel, the nest comprises a flat plate member and a tubular member, the flat plate member has at least one opening, and the tubular member has one end which is connected to the opening and the other end protrudes upward as a protruding end, wherein
the syringe barrel to be held in the nest includes a tubular barrel section for accommodating an internal-use solution in the interior thereof, an injection nozzle section formed at one end of the barrel section, and a flange section formed at an opening section on the other end of the barrel section to project radially outward;
the barrel section includes an intermediate body part having an outer diameter which is smaller than the outer diameter of the injection nozzle section and a large diameter barrel section having an outer diameter which is equal to the outer diameter of the injection nozzle section, and the large outer diameter barrel section is formed between the flange section and the intermediate body part;
the inner diameter of the tubular member is set to be larger than the outer diameter of the large outer diameter barrel section and smaller than the outer diameter of the flange section; and
the flange section is contacted with the protruding end of the tubular member, and at least a portion of the barrel section is held inside the tubular member.

4. A package of a medical syringe barrel according to Claim 3, wherein
the axial length of the large outer diameter barrel section is 1/3 or less of the axial length of the barrel section.
